Europäisches Patentamt

European Patent Office

Office européen des brevets

(19)

(11) **EP 1 239 398 A2**

(12) **EUROPEAN PATENT APPLICATION**

(43) Date of publication:
**11.09.2002 Bulletin 2002/37**

(51) Int Cl.[7]: **G06F 19/00**

(21) Application number: **02075119.4**

(22) Date of filing: **16.01.2002**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **17.01.2001 US 262580 P**

(71) Applicant: **GLAXO GROUP LIMITED**
**Greenford, Middlesex UB6 ONN (GB)**

(72) Inventors:
  • **Keefer, Christopher E., Glaxo SmithKline**
    **Research Triangle Park, NC 27709 (US)**
  • **Young, Sidney Stanley, Glaxo SmithKline**
    **Research Triangle Park, NC 27709 (US)**
  • **Xia, Tai-he**
    **Montgomery, NJ 08502 (US)**

(74) Representative: **Stott, Michael John et al**
    **GlaxoSmithKline**
    **Corporate Intellectual Property (CN9.25.1)**
    **980 Great West Road**
    **Brentford, Middlesex TW8 9GS (GB)**

(54) **Method, system and computer program product for identifying conditional associations among structures in samples**

(57) Conditional associations among features in samples are identified by defining a matrix having rows that represent the samples and columns that represent the features. Each row-column position of the matrix has a first binary value if the sample that is associated with the row exhibits the feature that is associated with the column, and a second binary value if a sample that is associated with the row does not exhibit the feature that is associated with the column. Recursive partitioning then is performed for each column. In particular, for each column, the column is recursively partitioned relative to the remaining ones of the columns, to define a tree of conditional branches for the rows for each column. The collection of trees of conditional branches for the columns may be displayed and/or analyzed to identify conditional associations of interest. Continuous features, wherein each row-column position of the matrix has a value selected from a continuous range of values, also may be analyzed.

FIG. 2

EP 1 239 398 A2

## Description

## Background of the Invention

This invention relates to data processing systems, methods and computer program products, and more particularly to statistical data analysis systems, methods and computer program products.

During the course of performing research and development, massive amounts of data often are collected for a plurality of samples, also referred to as objects or subjects, where each sample can include a plurality of features, also referred to as characteristics or traits. Discrete features may be present, wherein the sample either does or does not possess the feature (binary feature), or a small number of discrete levels of the feature are present (n-ary feature). Continuous features also may be present wherein the sample may include a value from a continuous range of values of the feature.

For example, massive amounts of genomic data are now becoming available. In this genomic data, the samples may be biological tissue samples and the features may be genes. The genes that are expressed in that sample can characterize the biological sample. Generally, most genes are not expressed, but some genes are expressed to varying degrees. The level of expression of a gene can be coded, for example as zero if not expressed or weakly expressed, and as one if expressed or strongly expressed, to provide a discrete feature. Alternatively, the features may be continuous traits such as weight, hair loss and blood pressure, that may be characterized by a value selected from a continuous range of values.

In another example, the sample is a consumer, and the features are various items for purchase at a store. The consumer selects various items for purchase that are noted at check-out. Still another example may relate to traffic flow, wherein a network, such as a road network or communications network includes multiple paths between nodes. The samples may be samples of vehicular or communications traffic between the nodes, and the features may be the various pathways in the network.

In all of the above and other examples, the features and samples may be related using a data table or matrix wherein, for example, the rows represent the plurality of samples and the columns represent the plurality of features. For discrete binary features, each row-column position of the matrix has a first binary value, for example 1, if the sample that is associated with the row exhibits a feature that is associated with a column, and a second binary value, for example 0, if the sample that is associated with the row does not exhibit the feature that is associated with the column. Thus, for example, an expressed gene in a sample can be indicated by a 1 at the position corresponding to the row of the sample and the column of the gene. Similarly, the purchasing of an item in a store by an individual can be represented by a 1 in the row-column position corresponding to the row of the individual and the column of the item. For continuous features, these 1s and 0s may be replaced by a value, preferably a scaled value, that indicates the value of the conditional feature.

It will be understood that, as used herein, the terms "row" and "column" indicate different directions in a matrix rather than absolute horizontal and vertical directions, and therefore may be interchanged. Moreover, it also will be understood that the term "matrix" is used to indicate any two-dimensional data structure that can represent features and samples, and may be represented in a data processing system as a table, database, memory map, linked list and/or other conventional representations. Specifically, conventional programming techniques may be used to store the data in a compact way and/or in a manner that can facilitate computation. Thus, for example, the data may be stored by column.

In the above and many other examples, the number of features can be quite large, for example on the order of hundreds, thousands or more. However, the number of features that are actually exhibited, represented by 1s, may typically be quite low. Moreover, many samples, on the order of hundreds, thousands or more, may be measured. The result may be a large, sparse data table or matrix.

In such large, sparse matrices, it often is desirable to determine associations among the features. For example, it is often desirable to determine which genes are expressed together, or which items are purchased together. The search for associations may be computationally-intensive. For example, for 1,000 columns and 500 rows, there may be approximately 500,000 pair-wise associations, and over 166,000,000 three-way associations.

In research and development activities, a determination of these associations may be highly desirable. Thus, for example, in a drug discovery and/or chemical synthesis process, there may be interest in determining which genes are expressed together or which molecular features occur together. In consumer marketing, there may be a desire to determine which items are purchased together. Accordingly, techniques have been developed to identify associations among features in samples. Examples from the pharmaceutical discovery field now will be described.

For example, in Walker et al., *Pharmaceutical Target Discovery Using Guilt-by-Association: Schizophrenia and Parkinson's Disease Genes,* Proceedings of the Seventh International Conference on Intelligent Systems for Molecular Biology, 1999, pp. 281-285, genes associated with a disease are identified by looking for novel genes whose expression patterns mimic those of known disease-associated genes. This method is referred to as "Guilt-by-Association" (GBA). As described in Walker et al., GBA uses a combinatoric measure of association that provides superior results to those from correlation measures used in previous expression analyses. Using GBA, the expression of 40,000 human genes in 522 cDNA libraries was examined, and several hundred genes associated with known cancer, inflammation, steroid-synthesis, insulin-synthesis, neurotransmitter processing, matrix remodeling and other disease genes were identified. See the Walker et al. abstract.

Other techniques for identifying associations among features display the matrix, for example using different colors to represent the value of the discrete or continuous features. See, for example, Alizadeh et al., *Distinct Types of Diffuse Large B-Cell Lymphoma Identified by Gene Expression Profiling,* Nature, Vol. 403, February 3, 2000, pp. 503-511. As described therein, for example, at Page 504, about 1.8 million measurements of gene expression were made in 96 normal and malignant lymphocyte samples using 128 Lymphochip microarrays. A hierarchical clustering algorithm was used to group genes on the basis of similarity in the pattern, with their expression varied over all samples. The data are shown in a matrix format. To visualize the result, the expression level of each gene relative to its median expression level across all samples was represented by a color, with red representing expression greater than the mean, green representing expression less than the mean, and the color intensity representing the magnitude of the deviation from the mean. Also see Hughes et al., *Functional Discovery Via A Compendium Of Expression Profiles,* Cell, Vol. 102, July 2000, pp. 109-126, at Page 118.

## Summary of the Invention

Embodiments of the invention identify conditional associations among a plurality of features in a plurality of samples, by defining a matrix having a plurality of rows that represent the plurality of samples and a plurality of columns that represent the plurality of features. Each row-column position of the matrix has a first binary value if the sample that is associated with the row exhibits the feature that is associated with the column, and a second binary value if a sample that is associated with the row does not exhibit the feature that is associated with the column. Recursive partitioning then is performed for each column. In particular, for each column, the column is recursively partitioned relative to the remaining ones of the columns, to define a tree of conditional branches for the rows for each column. The collection of trees of conditional branches for the columns may be displayed and/or analyzed to identify the conditional associations of interest.

As used herein, a "sample" is the source from which the values that populate the matrix are obtained. Samples may be biological or non-biological. A biological sample may be an entire organism (i.e., an animal, human, plant or microorganism). A biological sample may also be a cell, a plurality of cells, tissue, or fluid of an organism. The biological sample may be present in an organism (i.e., an *in situ* sample), or may be external to the organism. When external to the organism, the sample may be collected directly from an organism (i.e., as a tissue sample or as a bodily fluid such as blood, urine, plasma, or ascites), or may be removed from an organism and then optionally modified (i.e., by recombinant techniques), and/or stored (i.e., as a frozen sample), and/or maintained (i.e., in cell or tissue culture) away from the source organism for any period of time. A biological sample may also comprise one or more biological molecules or fragments thereof, including but not limited to one or more of a protein, polypeptide, enzyme, antibody, gene, nucleic acid, carbohydrate, or polysaccharide.

An individual sample may have a plurality of features that are characterized by values in the matrix. For example, when a biological sample is a whole organism, the features of the sample may be characteristics or traits of the organism. In the case of a human such traits may include, for example, hair color, age, sex, or presence/absence of disease. As another example, when the sample is a cell line, the features may include, for example, particular genes that are or are not expressed in the cell line. As still another example, when the biological sample is a gene, the features may include, for example, the expression of the gene in a plurality of tissue types or disease states.

In contrast with conventional methods of identifying associations among features in samples, embodiments of the present invention can find trees of conditional associations. For example, progressive subsets of samples where features are co-expressed may be found. This can provide rich data sets that can be used to analyze interactions among many features, and thereby allow deduction of pathways that can explain the interactions among the features. In gene expression profiling, embodiments of the invention can detect conditional associations among groups of genes rather than merely identifying pairs of genes that may be associated with one another. By identifying groups of genes that are conditionally associated, possible drug discovery programs and targets related to an identified susceptibility gene may be obtained. For example, if one gene in a group of genes is associated with a disease, then the other genes also may be associated.

Recursive partitioning may be performed, according to embodiments of the invention, by performing the following for each column. For the column, a number of occurrences of the first binary value in both the column and in each of the remaining columns is compared, to define a score for each of the remaining columns. One of the remaining columns is selected based on the scores. The rows that are associated with the selected column are divided based on whether the first value or the second value is present in the rows, to thereby obtain two sub-matrices and two corresponding branches of a tree. The above-described comparing, selecting and dividing is repeatedly performed for the rows of each sub-matrix, to obtain remaining branches of the tree. Accordingly, those features that have conditional associations can generate multi-level trees which can be used to identify conditional associations of features in the samples.

In embodiments of the invention, the one of the remaining columns may be selected based on a score by selecting one of the remaining columns that has a maximum score. The score may be defined using one or more statistical methods, such as chi-square, likelihood ratio and/or measure of agreement metrics. Moreover, auxiliary information concerning the samples may be used to aid in the selection. For example, in gene expression profiling, auxiliary information can include age, sex, hair color or other characteristics that form a basis for excluding some of the samples.

Moreover, in other embodiments, the repeated performance of the comparing, selecting and dividing may take place until a predefined termination is reached. The predefined termination may be determined by the scores being less than a predetermined score, the number of rows in a submatrix being less than a predetermined number, a tree reaching a predetermined depth and/or other tests.

The above-described embodiments have described discrete features where a feature either is present or absent in a sample. However, embodiments of the invention also may be used with continuous features, wherein each row-column position of the matrix has a value selected from a continuous range of values, which indicates an amount that the sample that is associated with the row exhibits a feature that is associated with the column. When performing recursive partitioning, the rows that are associated with the one of the remaining columns may be divided based on two or more range partitions of the values in the rows, to thereby obtain at least two submatrices and at least two corresponding branches of a tree. The trees of conditional branches may be displayed and/or analyzed, the scoring methodology may be selected and/or termination may be selected as was described above with regard to discrete features.

Accordingly, systems, methods and/or computer program products according to embodiments of the present invention can identify associations among a plurality of features in a plurality of samples by generating at least two trees of conditional branches for a corresponding at least two of the features. Each tree containing conditional branches indicates conditional associations for a corresponding feature relative to remaining ones of the plurality of features. It will be understood that embodiments of the invention may be provided as systems, methods and/or computer program products.

## Brief Description of the Drawings

Figure 1 is a block diagram of data processing systems according to embodiments of the present invention.

Figure 2 is a flowchart of methods, systems and/or computer program products for identifying conditional associations among a plurality of features in a plurality of samples according to embodiments of the present invention.

Figures 3A and 3B are examples of matrices that may be used when measuring discrete, binary or continuous features according to embodiments of the present invention.

Figure 4 illustrates a plurality of trees of conditional branches according to embodiments of the present invention.

Figure 5 is a flowchart of methods, systems and/or computer program products for recursive partitioning according to embodiments of the present invention.

Figure 6 illustrates a matrix including selection of a feature from among a plurality of features, according to embodiments of the present invention.

Figure 7 illustrates a 2 x 2 association table that may be used to compute a score according to embodiments of the present invention.

Figure 8 illustrates a matrix that includes auxiliary information according to embodiments of the present invention.

Figure 9 illustrates a matrix that is partitioned into submatrices according to embodiments of the present invention.

Figures 10A and 10B illustrate example trees of conditional data that may be generated using embodiments of the present invention.

## Detailed Description of Preferred Embodiments

The present invention now will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. Like numbers refer to like elements throughout.

The present invention is described below with reference to block diagrams and/or flowchart illustrations of methods, apparatus (systems) and/or computer program products according to embodiments of the invention. It is understood that each block of the block diagrams and/or flowchart illustrations, and combinations of blocks in the block diagrams and/or flowchart illustrations, can be implemented by computer program instructions. These computer program instructions may be provided to a processor of a general purpose computer, special purpose computer, and/or other programmable data processing apparatus to produce a machine, such that the instructions, which execute via the processor of the computer and/or other programmable data processing apparatus, create means for implementing the functions specified in the block diagrams and/or flowchart block or blocks.

These computer program instructions may also be stored in a computer-readable memory that can direct a computer or other programmable data processing apparatus to function in a particular manner, such that the instructions stored in the computer-readable memory produce an article of manufacture including instructions which implement the function specified in the block diagrams and/or flowchart block or blocks.

The computer program instructions may also be loaded onto a computer or other programmable data processing apparatus to cause a series of operational steps to be performed on the computer or other programmable apparatus to produce a computer implemented method such that the instructions which execute on the computer or other programmable apparatus provide steps for implementing the functions specified in the block diagrams and/or flowchart block or blocks.

It should also be noted that in some alternative implementations, the functions noted in the blocks may occur out of the order noted in the flowcharts. For example, two blocks shown in succession may in fact be executed substantially concurrently or the blocks may sometimes be executed in the reverse order, depending upon the functionality involved.

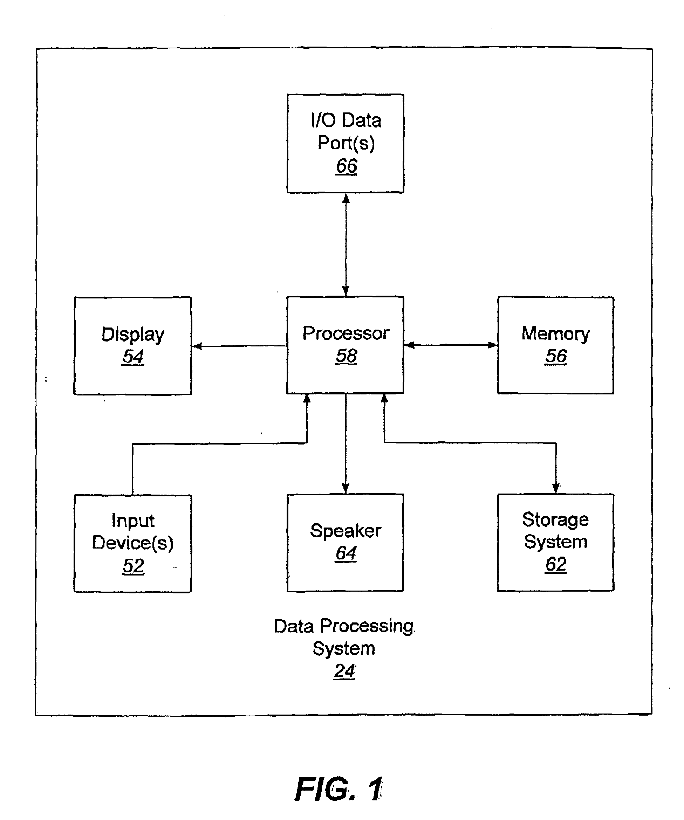

The present invention may be embodied in a data processing system such as illustrated in Figure 1. The data processing system 24 may be configured with computational, storage and control program resources for identifying conditional associations among a plurality of features in a plurality of samples, in accordance with embodiments of the present invention. Thus, the data processing system 24 may be contained in one or more enterprise, personal and/or pervasive computing devices, which may communicate over a network that may be a wired and/or wireless, public and/or private, local and/or wide area network such as the World Wide Web and/or a sneaker network using portable media. Moreover, when integrated into a single computing device, communication may take place via an Application Program Interface (API).

Still referring to Figure 1, embodiments of the data processing system 24 may include input device(s) 52, such as a keyboard or keypad, a display 54, and a memory 56 that communicate with a processor 58. The data processing system 24 may further include a storage system 62, a speaker 64, and an input/output (I/O) data port(s) 66 that also communicate with the processor 58. The storage system 62 may include removable and/or fixed media, such as floppy disks, ZIP drives, hard disks, or the like, as well as virtual storage, such as a RAMDISK. The I/O data port(s) 66 may be used to transfer information between the data processing system 24 and another computer system or a network (e.g., the Internet). These components may be conventional components such as those used in many conventional computing devices, which may be configured to operate as described herein.

The memory 56 may include an operating system to manage the data processing system resources and one or more applications programs including one or more application programs for identifying conditional associations among a plurality of features in a plurality of samples, according to embodiments of the present invention.

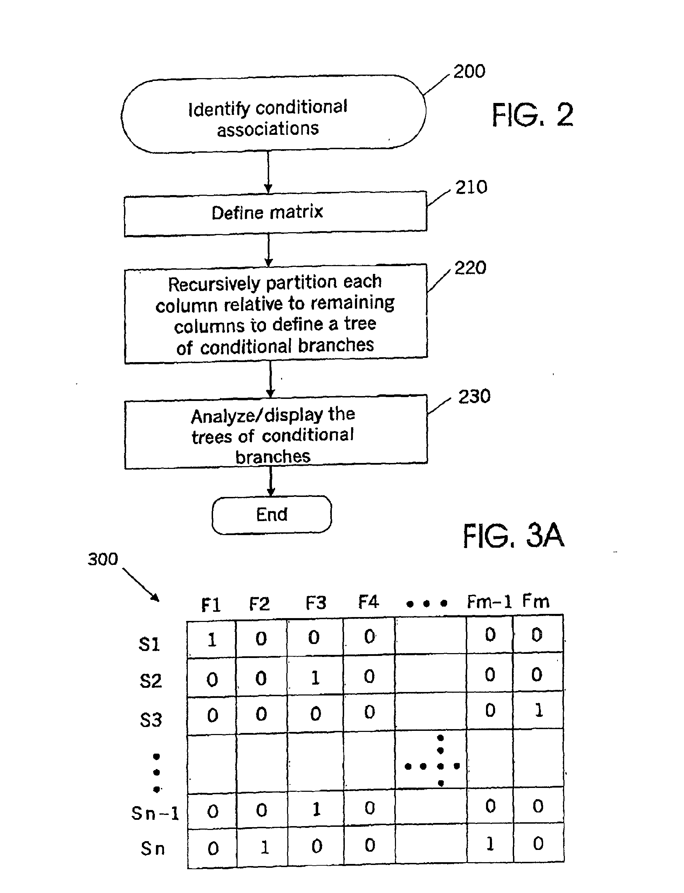

Figure 2 is a flowchart of methods, systems and/or computer program products 200 for identifying conditional associations among a plurality of features in a plurality of samples according to embodiments of the present invention. It will be understood that these systems, methods and/or computer program products 200 may be stored in a memory 56 of Figure 1 and may execute on the processor 58 of Figure 1.

Referring now to Figure 2, at Block 210, a matrix is defined having a plurality of rows that represent a plurality of samples and a plurality of columns that represent the plurality of features. In a drug discovery process, the samples may be a large number of samples, for example up to 10,000 or more samples from individual humans or organisms, and up to 10,000 or more genes and/or other features, that are measured in a sample. The samples and the features may be obtained using preexisting databases, clinical trials, microarray chips and/or many other conventional techniques.

As was described above, as used herein, the terms "row" and "column" indicate different directions in a matrix rather than absolute horizontal and vertical directions, and therefore may be interchanged. Moreover, the term "matrix" is used to indicate any two-dimensional structure that can represent features and samples, and may be represented in a data processing system as a table, database, memory map, linked list and/or other conventional representations.

Figure 3A is an example of a matrix 300 that may be used when measuring discrete, binary or continuous features. The rows of the matrix 300 comprise a plurality of samples S1-Sn, and the columns of the matrix comprise a plurality of features F1-Fm. For a binary discrete feature, such as disease present/absent or gene is expressed/is not expressed, each row-column position of the matrix has a first binary value, such as binary 1 if the sample that is associated with the row exhibits a feature that is associated with the column, and a second binary value, such as 0, if the sample that is associated with the row does not exhibit the feature that is associated with the column. Since conventionally, very few of the row-column intersections will exhibit the feature, the matrix of Figure 3A generally defines a "sparse" matrix.

Figure 3B illustrates an example of a matrix **310** that can be used when measuring a continuous feature, where each row-column position of the matrix **310** has a value selected from a range of continuous values that indicates an amount that the sample associated with the row exhibits the feature that is associated with the column. These values preferably are scaled so that they all fall within a given range. These values also can be treated to produce a sparse data matrix. For example, if the values are continuous, then if the value of the observation is extreme, it can be coded as 1. If not extreme, it can be coded as 0. Increases, decreases or both may be coded as 1. Extreme can be considered the extreme 5% or 10% of the distribution. Thus, a sparse matrix can be produced.

Referring again to Figure 2, at Block **220**, each column is recursively partitioned relative to the remaining columns, to thereby define a tree of conditional branches. Recursive partitioning is described, for example, in published PCT Application No. WO 98/47087, published 22 October 1998, to Farmen et al., entitled *Statistical Deconvoluting of Mixtures,* the disclosure of which is hereby incorporated herein by reference, and in a publication to Rusinko, III et al., entitled *Analysis of a Large Structure/Biological Activity Data Set Using Recursive Partitioning*, Journal of Chemical Information and Computer Science, Vol. 39, No. 6, 1999, pp. 1017-1026, the disclosure of which is incorporated herein by reference. Embodiments of recursive partitioning according to the present invention also will be described in detail below. Recursive partitioning can define a tree of conditional branches for the rows for each column. However, other techniques also may be used to define a tree of conditional branches for the rows for each column.

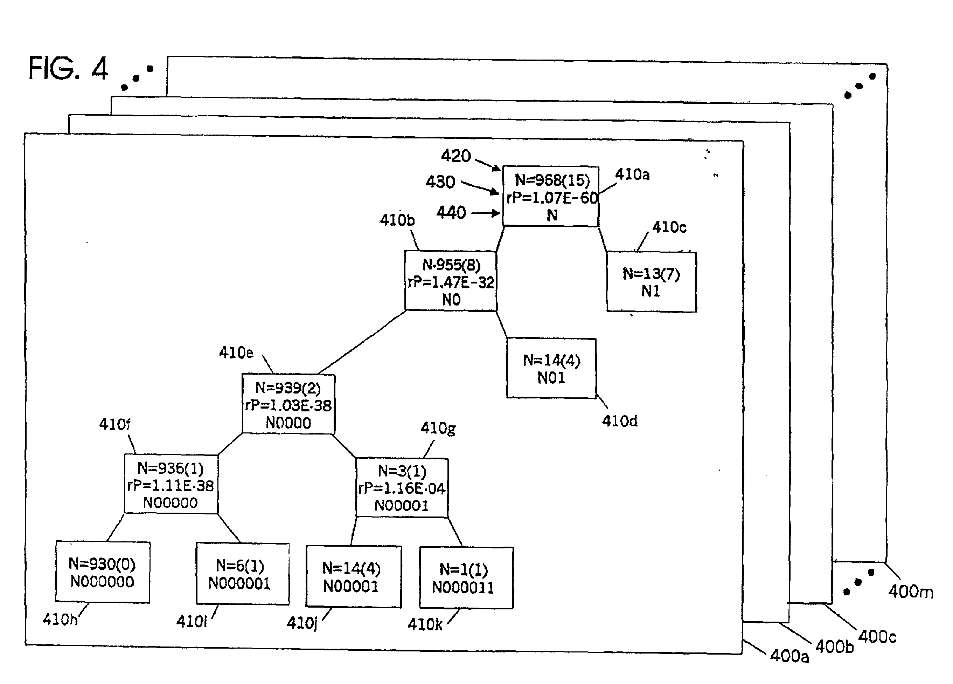

Figure 4 illustrates a plurality of trees **400a-400m** and an example of a tree **400a** of conditional branches for the rows of a particular column **Fj** when measuring a binary, discrete or continuous feature using a matrix **300** of, for example, Figure 3A. In Figure 4, the tree **400a** has eleven nodes **410a-410k**, and nodes **410a, 410b, 410e, 410f** and **410g** have two branches emanating therefrom, because a binary feature is being investigated. A ternary discrete feature may have three branches, a quaternary discrete feature may have four branches, etc. For continuous features, for example using a matrix **310** of Figure 3B, two or more branches may emanate from each node, depending upon how the range of continuous values is partitioned.

Referring again to Figure 4, each node **410a-410k** can include three lines of data associated therewith. The first line **420** identifies the total number of rows (samples) that are associated with the column (feature). Moreover, in the first line **420**, the number in parenthesis identifies the number of 1 s in that column. For example, in node **410a**, 968 rows are included, of which 15 have a value of 1.

Still referring to Figure 4, the second line **430** indicates a raw probability (rP) value which indicates a strength of a score for that node. Finally, the third line **440** provides an identification number for the node, so that the node can be associated with a column (feature). Other information may be included for each node. For example, an average value of the feature, a standard deviation and/or a standard error may be displayed. An adjusted probability value also may be used in addition to or instead of the raw probability value. For example, the raw probability value may be adjusted to reflect multiple testing. Many other display and/or printing formats for some or all of this and/or other data may be provided.

Additional details on the generation of the tree of conditional branches will be described below. However, it will be understood that a tree **400a-400m** may be generated for each column (feature) **F1-Fm**. Some of the trees may only include two levels, because there is not a large amount of conditional association. Others of the trees, for example the tree **400a** shown in Figure 4, will contain a more complex structure of nodes and branches, indicating the rich number of conditional associations. These complex trees may be analyzed in order to determine, for example, conditional associations among various genes that may be responsible for a given disease, as will described in detail below.

Referring again to Figure 2 at Block **230**, the trees of conditional branches, for example as shown in Figure 4, may be displayed on a display and/or in printed form, and/or analyzed using human and/or machine analysis.

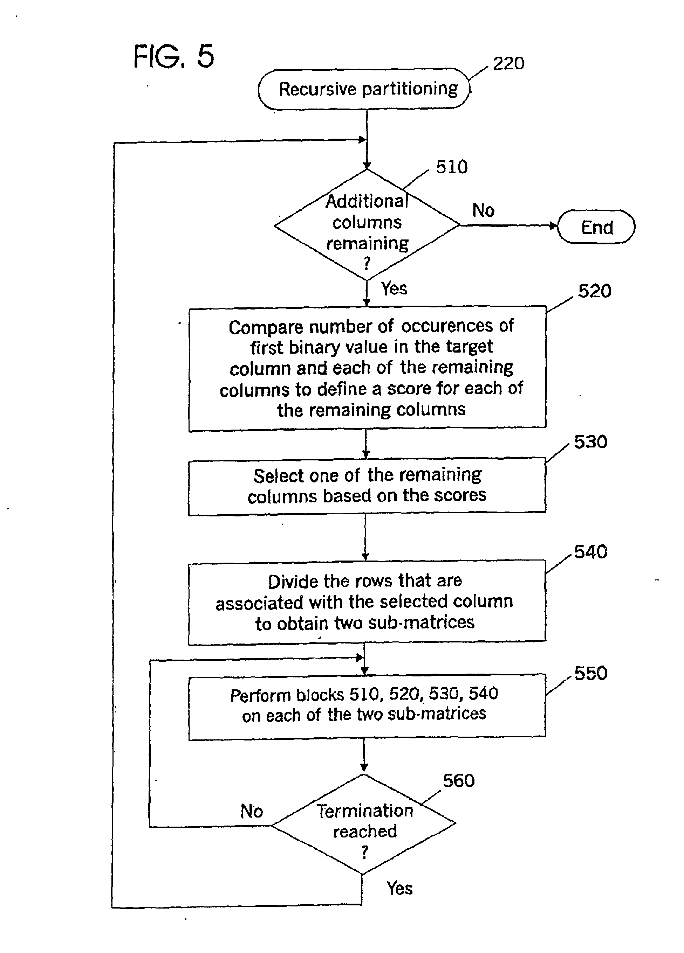

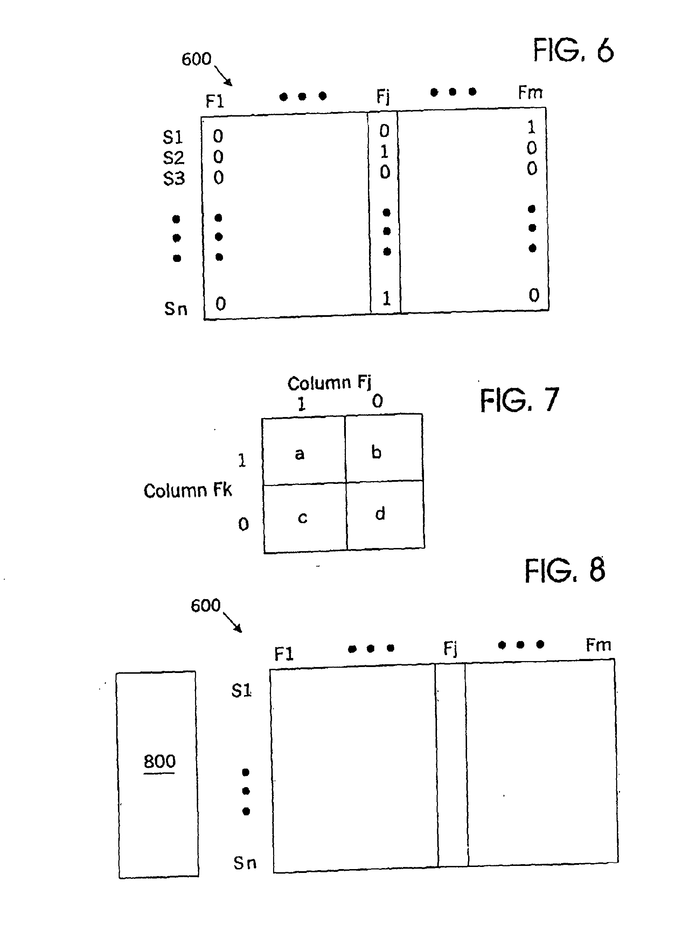

Referring now to Figure 5, a flowchart illustrating recursive partitioning **220** according to embodiments of the present invention now will be described. As shown in Figure 5, recursive partitioning **220** begins by selecting a column **Fj** of a matrix, such as a matrix **300** of Figure 3A. Referring now to Block **510,** the operations of Figure 5 are performed on each column of the matrix by selecting a feature **Fj** from features **F1-Fm**, where j preferably is sequentially selected from l to m, so that the operations are performed for each column. Figure 6 diagramatically illustrates the selection of a column **Fj** from among the columns **F1-Fm** in a matrix **600.** As described above, the selected column typically will iterate from the first column **F1** until the last column **Fm** is processed.

Referring again to Figure 5, at Block **520,** a score is computed for each of the remaining columns **Fk**, where k≠j, relative to the selected column **Fj**. The score may be computed by comparing a number of occurrences of the first binary value (1) in both the selected column **Fj** and each of the remaining columns **Fk**, where $k \neq j$. In some embodiments, the score may be computed by creating a 2 x 2 association table **700** shown in Figure 7. As shown in Figure 7, when comparing column **Fj** and column **Fk**, each occurrence of a 1 in both column **Fj** and in column **Fk** increments the count a. Each occurrence of a 0 in column **Fj** and a 1 in column **Fk** increments the count b. Each occurrence of a 1 in column **Fj** and a 0 in column **Fk** increments the count c. Each occurrence of a 0 in a column **Fj** and a 0 in column **Fk** increments the count d. It will be understood that the sum of a + b + c + d equals the number of samples n. A 2 x 2 association table **700** may be determined for each comparison of the selected column **Fj** with each of the remaining columns **Fk**. The score that is associated with each of the columns in the comparison may be the number of occurrences of the value 1 in both columns **Fj** and **Fk**, in other words, the value of a. Alternatively, more sophisticated probability functions may be used to determine a score for each column. For example, a chisquare ($\Pi^2$) test may be used, wherein

$$K^{x^2} = \frac{|ad - bc|^2 n}{(a + b)(c + d)(a + c)(b + d)}.$$

As is well known to those having skill in the art, the $\Pi^2$ test may be used to measure an amount of association in a 2 x 2 association table. Moreover, the result of the $\Pi^2$ test may be converted into a probability value P, using well known techniques.

It also will be understood that other statistical tests maybe used to compute a score for each of the remaining columns relative to the selected column. A Shannon information test, Fisher exact test, Fisher exact test modified to discount a 00 cell, Pearson correlation coefficient and/or other conventional statistical test may be used to establish a score for discrete or continuous functions. These and other statistical tests are described, for example, in a publication to An et al., entitled *Rule Quality Measures Improve the Accuracy of Rule Induction. An Experimental Approach,* 12th International Symposium on Methodologies for Intelligent Systems, Charlotte, NC, 2000, pp. 119-129, the disclosure of which is hereby incorporated herein by reference.

Referring again to Figure 5, at Block **530**, one of the remaining columns **Fk** is selected based on the scores. For example, in one embodiment, the column **Fk** that has the highest score is selected. This selection may be performed automatically by the data processing system **24** without human interaction. Moreover, the one of the remaining columns **Fk** that is selected may be selected based on the scores and based upon auxiliary information. The auxiliary information may be used by an expert to select one of the remaining columns and/or may be processed automatically without human interaction.

For example, Figure 8 illustrates the addition of auxiliary information **800** for each of the samples **S1-Sn** in the matrix **600.** The auxiliary information **800** may include external characteristics such as sex, age, hair color and/or presence/absence of a disease, and may be used to impact the selection of the samples to be used. In a specific example, for a particular drug investigation, associations only among samples that are women may be desired. Alternatively associations only among samples that have the disease may be desired. Thus, this auxiliary information concerning the samples may be used in addition to the score, to select appropriate samples **Fk** and/or to determine the scores.

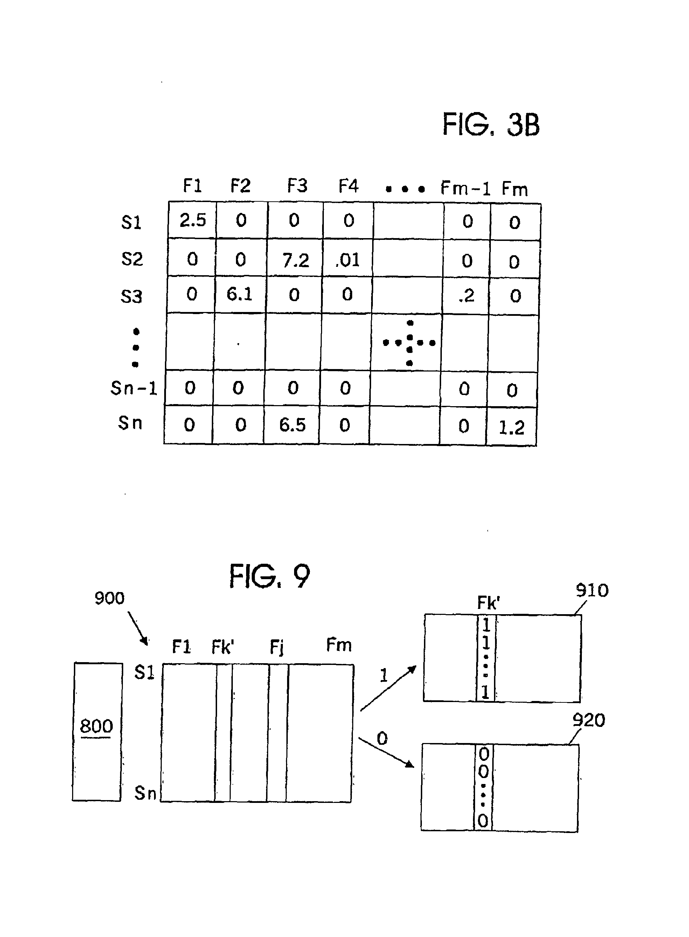

Referring again to Figure 5, at Block **540**, the rows that are associated with the selected column are then divided to obtain two submatrices. Embodiments of these operations are illustrated with reference to Figure 9. Figure 9 illustrates a matrix wherein a selected column **Fj** is compared with all of the remaining columns **Fk,** and one of the remaining columns **Fk'** with the highest score is obtained. For the column **Fk',** all of the rows that have a 1 are collected, to thereby obtain a first submatrix **910,** and all the rows that have a 0 are collected to obtain a second submatrix **920**. It will be understood that submatrices **910** and **920** may be generated by rearranging the rows of the matrix **900** of Figure 9, so that all the 1s and all the 0s in column **Fk'** are grouped together. Alternatively, the matrices **910** and **920** may be generated by generating pointers to the various rows that have a 1 or a 0 in column **Fk'**. The rows in the submatrices **910** and **920** also may be constrained by the auxiliary information as described above.

Referring again to Figure 5, at Block **550**, the operations at Blocks **510**, **520**, **530** and **540** then are performed recursively on each of the two submatrices **910**, **920** to continue generating submatrices. These operations may be performed until a termination is reached at Block **560**.

Termination may be reached when satisfying one or more criteria. For example, operations for a given column may terminate when the number of branching levels in the tree exceeds a predetermined depth, for example when the tree is more than ten levels deep. Alternatively, the number of rows in a submatrix **910** or **920** may be less than a predetermined number. In other alternatives, the scores may be less than a predetermined score. Combinations of these criteria and/or other criteria may be used to define termination. Upon termination, a second column **Fj** is selected at Block **510**, and operations continue for this column until all columns have been recursively partitioned.

Thus, referring again to Figure 4, for a column corresponding to any given feature, a tree, such as tree **400a**, may be generated. The topmost node **410a** of the tree **400a** represents the selected feature **Fk**. The two nodes **410b** and **410c** that branch from the top node **410a** represent a feature **Fk'** having the second value (0) and the first value (1) respectively. In other words, in Figure 4, 0s branch to the left and 1s branch to the right. It will be understood that other embodiments may branch 1 s to the left and 0s to the right.

Still referring to Figure 4, at Block **410a**, for a selected column **Fk** having 968 samples and 15 samples with a 1 value, a split was performed to a first submatrix **910** corresponding to node **410c** having thirteen samples of which seven are 1s, and a second submatrix **920** corresponding to node **410b**, having 955 values of which eight are 1s. Termination was reached for node **410c,** so that no further processing was performed. However, node 410b again was partitioned into node **410e** and **410d**. Partitioning continues until termination is reached for all of nodes **410h**, **410i**, **410j** and **410k.**

It will be understood that the operations of Figure 5 have been described primarily with respect to binary or n-ary discrete features. For continuous features, the associations between a selected column and the remaining columns of Block **520** may be obtained, for example, using segmentation calculations rather than the association table of Figure 7, to thereby define a score. Dividing the rows into two or more groups of rows is referred to as the segmentation or change-point problem and is described, for example, in Venter et al., *Finding Multiple Abrupt Change Points,* Computational Statistics and Data Analysis, Vol. 22, No. 5, 1996, pp. 481-504. One of the remaining columns may be selected based on the scores, and the rows that are associated with the selected column may be divided or partitioned based on range partitions of the continuous values in the rows, to thereby obtain at least two submatrices and at least two corresponding branches of the tree. The range partitions may be selected, for example, by generating a scatter plot that can identify appropriate clustering ranges and/or by segmentation techniques. Thus, for continuous features, each node may branch into two, three or more branches depending upon the number of range partitions of the continuous values that is desired. The operations otherwise can be similar to those described in connection with Figure 5.

Conventional "Guilt-by-Association" methods, as exemplified in the above-cited Walker et al. publication, can identify an association among a specific target gene and other genes. In contrast, embodiments of the present invention can identify conditional associations among genes that are associated with a target. Thus, for example, if gene 1 is associated with the target, embodiments of the present invention can find genes associated with gene 1 as well, to thereby obtain a tree of conditional associations. Moreover, the Walker et al. publication uses a Fisher exact test that considers all of the cells of a 2 x 2 association table. Embodiments of the present invention can use statistical methods that focus on the 1, 1 cell; i.e., genes that are coexpressed. Thus, attention may be focused on co-expression rather than co-nonexpression. For example, if two individuals have red hair, they are similar in some sense. If two individuals do not have red hair, then that is less strong evidence that they are similar.

Embodiments of the present invention can use recursive partitioning trees to divide the rows of the data table successively into groups of rows. One column is selected. For all of the other columns in turn, a division is determined based upon the 0s and 1s in those columns. A score for each possible division is computed.

In an automatic mode, the column with the best score may be used to divide the rows into two groups: rows with a 1 in that column may be sent to the right daughter node and rows with a 0 may be sent to the left daughter node. In expert use mode, an expert may examine the scores and any auxiliary information on the subjects that are in the resulting nodes, and may select a column to use for the split. Thus, embodiments of the present invention can identify all possible association pairs, using each column as a target column in turn.

Embodiments of the present invention may be used to test associations of the expression of genes in human or other biological tissues. Embodiments of the invention also may be used to test associations of consumer purchases, the so-called "market basket" problem. Embodiments of the present invention also may be used to determine associations among routes between nodes in a network, such as a communication or highway network, the so-called "traffic flow" problem. Other examples of associations of a large number of features to a large number of samples may be analyzed.

### Example

The following example is illustrative and shall not be construed as limiting the scope of the present invention.

In a publication by Alizadeh et al., entitled *Distinct Types of Diffuse Large B-Cell Lymphoma Identified by Gene Expression Profiling*, Nature, Vol. 403, February 2000, pp. 503-511, gene expression in normal and malignant lymphocyte samples is reported. An assay of 128 samples and over 17,000 genes was performed. Some of the samples were duplicates and some of the genes had low levels of expression. A data set of 96 non-duplicate samples and gene expressions of 4026 was made available in the supplemental material that accompanied the Alizadeh et al. publication.

The Alizadeh et al. data set was recoded as follows: Each of the 4026 genes was examined and the expression level was coded as 1 if the expression level was in the top 10% of the distribution. Otherwise, the expression level was coded as 0. Up-regulated genes were considered. One of these genes, gene 3755, was selected as the target gene to predict. The remaining gene levels, i.e. 4025 genes coded as 1 or 0, were used to predict the 1/0 expression level of gene 3755.

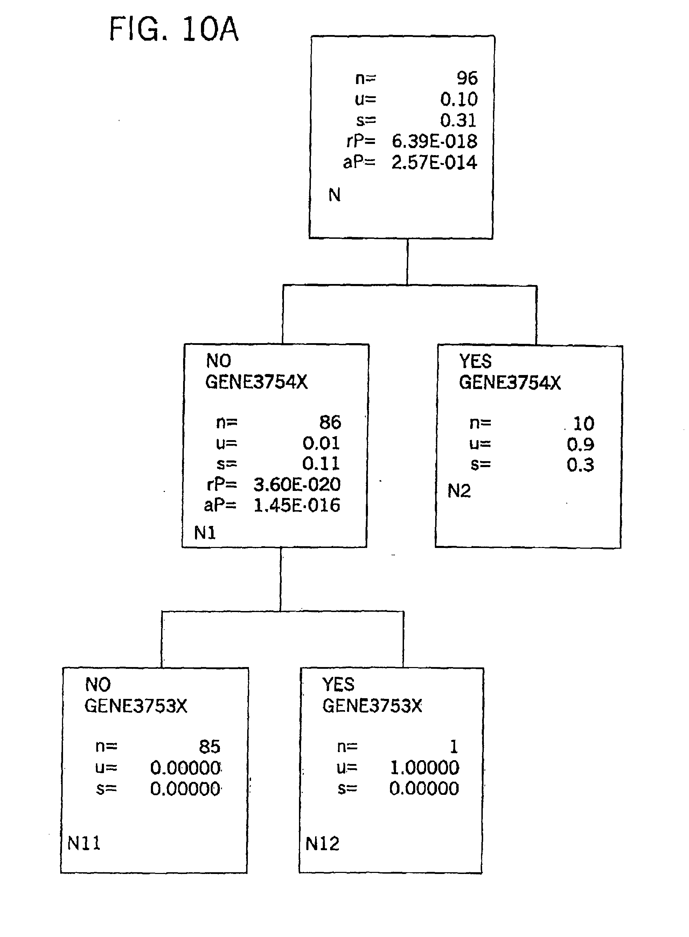

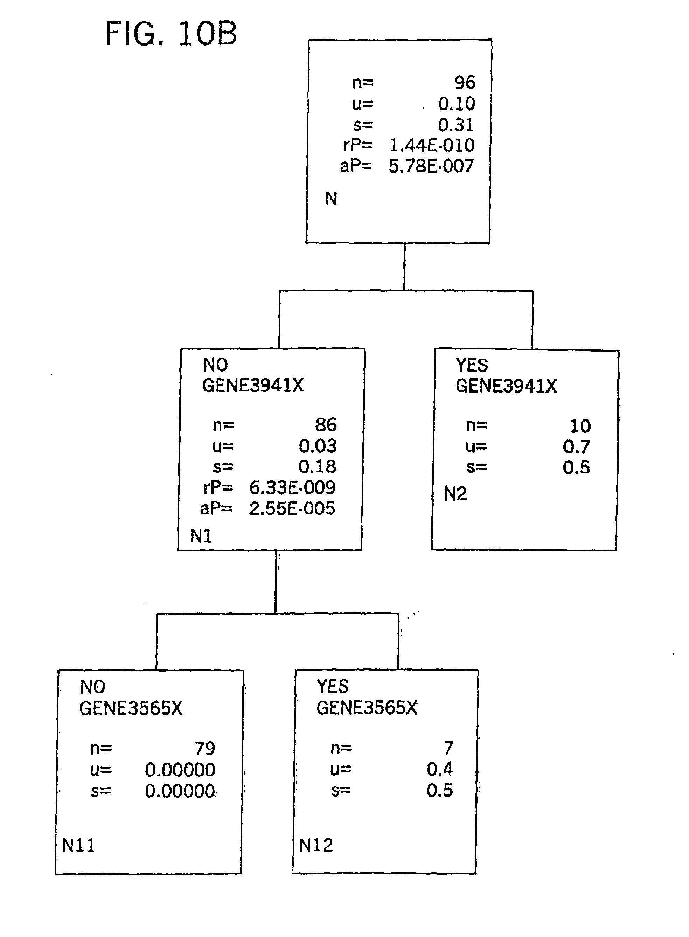

Figures 10A and 10B illustrate two recursive partitioning trees that were generated. Referring now to Figure 10A, genes 3754 and 3753 were used to split the data and predict gene 3755. These genes are highly related to the target gene and, although predictive, they may be of relatively little interest. The two predictive genes, genes 3754 and 3753, are detected as highly correlated with gene 3755. They are predictive without conditioning on other genes.

In Figure 10B, genes 3941 and 3565 are used to split the data and predict gene 3755. Gene 3941 is used to make the first split. Seven of the ten samples showing high expression of gene 3755 were found. In these samples without high expression of gene 3941, i. e. 86 samples, high levels of expression of gene 3565 are able to find three additional samples with a high level of gene 3755.

Accordingly, the importance of gene 3565 was not detected at the first split. It was detected as being important only after tissues expressing gene 3941 were removed from the data set, and is conditional on not expressing gene 3941 in an up-regulated state. Gene 3565 therefore was not detected as being important in the entire data set, but was detected as being important, conditional on a low expression level of gene 3941.

Thus, if gene 3755 was a "guilty" gene, then genes 3754, 3753 and others would be guilty by association. These genes are directly associated with one another.

Gene 3565 also is guilty, but only conditionally, through a secondary association when gene 3941 is not up-regulated. Gene 3565 is predictive of gene 3755 when gene 3941 is not at a high level of expression. Gene 3565 was not determined to be important in the initial search over all the 4025 genes.

In the drawings and specification, there have been disclosed typical preferred embodiments of the invention and, although specific terms are employed, they are used in a generic and descriptive sense only and not for purposes of limitation, the scope of the invention being set forth in the following claims.

## Claims

1. A method of identifying conditional associations among a plurality of features in a plurality of samples, the method comprising:

   defining a matrix having a plurality of rows that represent the plurality of samples and a plurality of columns that represent the plurality of features, each row-column position of the matrix having a first binary value if the sample that is associated with the row exhibits the feature that is associated with the column and a second binary value if the sample that is associated with the row does not exhibit the feature that is associated with the column; and
   for each column, recursively partitioning the column relative to remaining ones of the columns to define a tree of conditional branches for the rows for each column.

2. A method according to Claim 1 wherein the recursively partitioning is followed by:

   analyzing the trees of conditional branches for the columns to identify the conditional associations.

   ations.

3. A method according to Claim 1 wherein the recursively partitioning is followed by:

   displaying the trees of conditional branches for the columns to identify the conditional associations.

4. A method according to Claim 1 wherein the recursively partitioning comprises the following that are performed for each column:

   for the column, comparing a number of occurrences of the first binary value in both the column and in each of the remaining columns to define a score for each of the remaining columns;
   selecting one of the remaining columns based upon the scores;
   dividing the rows that are associated with the one of the remaining columns based on whether the first value or the second value is present in the rows, to thereby obtain two sub-matrices and two corresponding branches of a tree; and
   repeatedly performing the comparing, selecting and dividing for the columns of each of the sub matrices that are associated with the two branches of the tree to obtain remaining branches of the tree.

5. A method according to Claim 4 wherein the selecting comprises selecting one of the remaining columns that has a maximum score.

6. A method according to Claim 4 wherein the selecting comprises selecting one of the remaining columns based upon the scores and auxiliary information concerning the samples.

7. A method according to Claim 4 wherein the repeatedly performing comprises repeatedly performing the comparing, selecting and dividing of the rows of each sub-matrix until a predefined termination is reached.

8. A method according to Claim 7 wherein the predefined termination comprises at least one of the scores in the remaining columns being less than a predetermined score, the number of rows in a sub-matrix being less than a predetermined number and the tree having a predetermined depth.

9. A method according to Claim 4 wherein the comparing a number of occurrences of the first binary value in both the column and in each of the remaining columns to define a score for each of the remaining columns comprises comparing a number of occurrences of the first binary value in both the column and in each of the remaining columns using at least one of a Pearson chi-square, likelihood ratio statistic and measure of agreement metric.

10. A method according to Claim 1 wherein the samples are biological samples, the features are genes, the first binary value indicates that the gene is expressed in the biological sample and the second binary value indicates that the gene is not expressed in the biological sample.

11. A method of identifying conditional associations among a plurality of features in a plurality of samples, the method comprising:

defining a matrix having a plurality of rows that represent the plurality of samples and a plurality of columns that represent the plurality of features, each row-column position of the matrix having a value selected from a continuous range of values that indicates an amount that the sample that is associated with the row exhibits the feature that is associated with the column; and
for each column, recursively partitioning the column relative to remaining ones of the columns to define a tree of conditional branches for the rows for each column.

12. A method according to Claim 11 wherein the recursively partitioning comprises the following that are performed for each column:

for the column, identifying an association between the column and each of the remaining columns to define a score for each of the remaining columns;
selecting one of the remaining columns based upon the scores;
dividing the rows that are associated with the one of the remaining columns based on range partitions of the values in the rows to thereby obtain at least two submatrices and at least two corresponding branches of a tree; and
repeatedly performing the comparing, selecting and dividing for the columns of each of the sub matrices that are associated with the at least two branches of the tree to obtain remaining branches of the tree.

13. A method according to Claim 12 wherein the selecting comprises selecting one of the remaining columns that has a highest correlation coefficient with the column.

14. A method according to Claim 11 wherein the samples are biological samples, the features are continuous traits of the biological samples and the value indicates an amount that the biological sample that is associated with the row exhibits the continuous trait.

15. A method of identifying associations among a plurality of features in a plurality of samples, the method comprising:

generating at least two trees of conditional branches for a corresponding at least two of the features, each tree of conditional branches indicating conditional associations for a corresponding feature relative to remaining ones of the plurality of features.

16. A method according to Claim 15 wherein the samples are biological samples and the features are discrete traits of the biological samples.

17. A method according to Claim 15 wherein the samples are biological samples and the features are continuous traits of the biological samples.

18. A system for identifying conditional associations among a plurality of features in a plurality of samples, the system comprising:

a matrix having a plurality of rows that represent the plurality of samples and a plurality of columns that represent the plurality of features, each row-column position of the matrix having a first binary value if the sample that is associated with the row exhibits the feature that is associated with the column and a second binary value if the sample that is associated with the row does not exhibit the feature that is associated with the column; and
means for recursively partitioning each column relative to remaining ones of the columns to define a tree of conditional branches for the rows for each column.

19. A system according to Claim 18 further comprising:

means for analyzing the trees of conditional branches for the columns to identify the conditional associations.

20. A system according to Claim 18 further comprising:

means for displaying the trees of conditional branches for the columns to identify the conditional associations.

21. A system according to Claim 18 wherein the means for recursively partitioning comprises:

means for comparing a number of occurrences of the first binary value in both the column and in each of the remaining columns to define a score for each of the remaining columns;

means for selecting one of the remaining columns based upon the scores;

means for dividing the rows that are associated with the one of the remaining columns based on whether the first value or the second value is present in the rows, to thereby obtain two sub-matrices and two corresponding branches of a tree; and

means for repeatedly activating the means for comparing, the means for selecting and the means for dividing for the columns of each of the sub matrices that are associated with the two branches of the tree to obtain remaining branches of the tree.

22. A system according to Claim 21 wherein the means for selecting comprises means for selecting one of the remaining columns that has a maximum score.

23. A system according to Claim 21 wherein the means for selecting comprises means for selecting one of the remaining columns based upon the scores and auxiliary information concerning the samples.

24. A system according to Claim 21 wherein the means for repeatedly activating comprises means for repeatedly activating the means for comparing, the means for selecting and the means for dividing for the rows of each sub-matrix until a predefined termination is reached.

25. A system according to Claim 24 wherein the predefined termination comprises at least one of the scores in the remaining columns being less than a predetermined score, the number of rows in a sub-matrix being less than a predetermined number and the tree having a predetermined depth.

26. A system according to Claim 21 wherein the means for comparing a number of occurrences of the first binary value in both the column and in each of the remaining columns to define a score for each of the remaining columns comprises means for comparing a number of occurrences of the first binary value in both the column and in each of the remaining columns using at least one of a Pearson chi-square, likelihood ratio statistic and measure of agreement metric.

27. A system according to Claim 18 wherein the samples are biological samples, the features are genes, the first binary value indicates that the gene is expressed in the biological sample and the second binary value indicates that the gene is not expressed in the biological sample.

28. A system for identifying conditional associations among a plurality of features in a plurality of samples, the system comprising:

a matrix having a plurality of rows that represent the plurality of samples and a plurality of columns that represent the plurality of features, each row-column position of the matrix having a value selected from a continuous range of values that indicates an amount that the sample that is associated with the row exhibits the feature that is associated with the column; and

means for recursively partitioning each column relative to remaining ones of the columns to define a tree of conditional branches for the rows for each column.

29. A system according to Claim 28 wherein the means for recursively partitioning comprises:

means for identifying an association between the column and each of the remaining columns to define a score for each of the remaining columns;

means for selecting one of the remaining columns based upon the scores;

means for dividing the rows that are associated with the one of the remaining columns based on range partitions of the values in the rows to thereby obtain at least two sub-matrices and at least two corresponding branches of a tree; and

means for repeatedly activating the means for comparing, the means for selecting and the means for dividing for the columns of each of the sub matrices that are associated with the at least two branches of the tree to obtain remaining branches of the tree.

30. A system according to Claim 29 wherein the means for selecting comprises means for selecting one of the remaining columns that has a highest correlation coefficient with the column.

31. A system according to Claim 28 wherein the samples are biological samples, the features are continuous traits of the biological samples and the value indicates an amount that the biological sample that is associated with the row exhibits the continuous trait.

32. A system for identifying associations among a plurality of features in a plurality of samples, the system comprising:

means for generating at least two trees of conditional branches for a corresponding at least two of the features, each tree of conditional branches indicating conditional associations for a corresponding feature relative to remaining ones of the plurality of features; and means for displaying the at least two trees of conditional branches.

33. A system according to Claim 32 wherein the samples are biological samples and the features are discrete traits of the biological samples.

34. A system according to Claim 32 wherein the samples are biological samples and the features are continuous traits of the biological samples.

35. A computer program product that identifies conditional associations among a plurality of features in a plurality of samples, the computer program product comprising a computer usable storage medium having computer-readable program code embodied in the medium, the computer-readable program code comprising:

computer-readable program code that is configured to define a matrix having a plurality of rows that represent the plurality of samples and a plurality of columns that represent the plurality of features, each row-column position of the matrix having a first binary value if the sample that is associated with the row exhibits the feature that is associated with the column and a second binary value if the sample that is associated with the row does not exhibit the feature that is associated with the column; and computer-readable program code that is configured to recursively partition each column relative to remaining ones of the columns to define a tree of conditional branches for the rows for each column.

36. A computer program product according to Claim 35 further comprising:

computer-readable program code that is configured to analyze the trees of conditional branches for the columns to identify the conditional associations.

37. A computer program product according to Claim 35 further comprising:

computer-readable program code that is configured to display the trees of conditional branches for the columns to identify the conditional associations.

38. A computer program product according to Claim 35 wherein the computer-readable program code that is configured to recursively partition comprises:

computer-readable program code that is configured to compare a number of occurrences of the first binary value in both the column and in each of the remaining columns to define a score for each of the remaining columns; computer-readable program code that is configured to select one of the remaining columns based upon the scores; computer-readable program code that is configured to divide the rows that are associated with the one of the remaining columns based on whether the first value or the second value is present in the rows, to thereby obtain two sub-matrices and two corresponding branches of a tree; and computer-readable program code that is configured to repeatedly activate the computer-readable program code that is configured to compare, the computer-readable program code that is configured to select and the computer-readable program code that is configured to divide for the columns of each of the sub matrices that are associated with the two branches of the tree to obtain remaining branches of the tree.

39. A computer program product according to Claim 38 wherein the computer-readable program code that is configured to select comprises computer-readable program code that is configured to select one of the remaining columns that has a maximum score.

40. A computer program product according to Claim 38 wherein the computer-readable program code that is configured to select comprises computer-readable program code that is configured to select one of the remaining columns based upon the scores and auxiliary information concerning the samples.

41. A computer program product according to Claim 38 wherein the computer-readable program code that is configured to repeatedly activate comprises computer-readable program code that is configured to repeatedly activate the computer-readable program code that is configured to compare, the computer-readable program code that is configured to select and the computer-readable program code that is configured to divide rows of each sub-matrix until a predefined termination is reached.

42. A computer program product according to Claim 41 wherein the predefined termination comprises at least one of the scores in the remaining columns being less than a predetermined score, the number of rows in a sub-matrix being less than a predetermined number and the tree having a predetermined depth.

43. A computer program product according to Claim 38 wherein the computer-readable program code that is configured to compare a number of occurrences of the first binary value in both the column and in each of the remaining columns to define a score for each of the remaining columns comprises computer-readable program code that is configured to compare a number of occurrences of the first binary value in both the column and in each of the remaining columns using at least one of a Pearson chi-square, likelihood ratio statistic and measure of agreement metric.

44. A computer program product according to Claim 35 wherein the samples are biological samples, the features are genes, the first binary value indicates that the gene is expressed in the biological sample and the second binary value indicates that the gene is not expressed in the biological sample.

45. A computer program product that identifies conditional associations among a plurality of features in a plurality of samples, the computer program product comprising a computer usable storage medium having computer-readable program code embodied in the medium, the computer-readable program code comprising:

> computer-readable program code that is configured to define a matrix having a plurality of rows that represent the plurality of samples and a plurality of columns that represent the plurality of features, each row-column position of the matrix having a value selected from a continuous range of values that indicates an amount that the sample that is associated with the row exhibits the feature that is associated with the column; and
> computer-readable program code that is configured to recursively partition each column relative to remaining ones of the columns to define a tree of conditional branches for the rows for each column.

46. A computer program product according to Claim 45 wherein the computer-readable program code that is configured to recursively partition comprises:

> computer-readable program code that is configured to identify an association between the column and each of the remaining columns to define a score for each of the remaining columns;
> computer-readable program code that is configured to select one of the remaining columns based upon the scores;

computer-readable program code that is configured to divide the rows that are associated with the one of the remaining columns based on range partitions of the values in the rows to thereby obtain at least two sub-matrices and at least two corresponding branches of a tree; and computer-readable program code that is configured to repeatedly activate the computer-readable program code that is configured to compare, the computer-readable program code that is configured to select and the computer-readable program code that is configured to divide for the columns of each of the sub matrices that are associated with the at least two branches of the tree to obtain remaining branches of the tree.

47. A computer program product according to Claim 46 wherein the computer-readable program code that is configured to select comprises computer-readable program code that is configured to select one of the remaining columns that has a highest correlation coefficient with the column.

48. A computer program product according to Claim 45 wherein the samples are biological samples, the features are continuous traits of the biological samples and the value indicates an amount that the biological sample that is associated with the row exhibits the continuous trait.

49. A computer program product that identifies associations among a plurality of features in a plurality of samples, the computer program product comprising a computer usable storage medium having computer-readable program code embodied in the medium, the computer-readable program code comprising:

> computer-readable program code that is configured to generate at least two trees of conditional branches for a corresponding at least two of the features, each tree of conditional branches indicating conditional associations for a corresponding feature relative to remaining ones of the plurality of features.

50. A computer program product according to Claim 49 wherein the samples are biological samples and the features are discrete traits of the biological samples.

51. A computer program product according to Claim 49 wherein the samples are biological samples and the features are continuous traits of the biological samples.

I/O Data
Port(s)
66

Display
54

Processor
58

Memory
56

Input
Device(s)
52

Speaker
64

Storage
System
62

Data Processing
System
24

FIG. 1

**FIG. 2**

- 200 — Identify conditional associations
- 210 — Define matrix
- 220 — Recursively partition each column relative to remaining columns to define a tree of conditional branches
- 230 — Analyze/display the trees of conditional branches
- End

**FIG. 3A**

300

| | F1 | F2 | F3 | F4 | • • • | Fm−1 | Fm |
|---|---|---|---|---|---|---|---|
| S1 | 1 | 0 | 0 | 0 | | 0 | 0 |
| S2 | 0 | 0 | 1 | 0 | | 0 | 0 |
| S3 | 0 | 0 | 0 | 0 | | 0 | 1 |
| ⋮ | | | | | • • • | | |
| Sn−1 | 0 | 0 | 1 | 0 | | 0 | 0 |
| Sn | 0 | 1 | 0 | 0 | | 1 | 0 |

# FIG. 4

## FIG. 5

```
                    ┌─────────────────────────┐  ╱ 220
                    │  Recursive partitioning  │
                    └─────────────────────────┘
                               │
                               ▼
                          ╱ 510
                        ╱  Additional ╲        No        ┌─────────┐
                       ◇   columns      ◇  ──────────▶   │   End   │
                        ╲  remaining  ╱                  └─────────┘
                         ╲    ?     ╱
                               │ Yes
                               ▼              ╱ 520
        ┌──────────────────────────────────────────────┐
        │  Compare number of occurences of             │
        │  first binary value in the target            │
        │  column and each of the remaining            │
        │  columns to define a score for each of       │
        │  the remaining columns                       │
        └──────────────────────────────────────────────┘
                               │
                               ▼              ╱ 530
        ┌──────────────────────────────────────────────┐
        │  Select one of the remaining                 │
        │  columns based on the scores                 │
        └──────────────────────────────────────────────┘
                               │
                               ▼              ╱ 540
        ┌──────────────────────────────────────────────┐
        │  Divide the rows that are                    │
        │  associated with the selected column         │
        │  to obtain two sub-matrices                  │
        └──────────────────────────────────────────────┘
                               │
                               ▼              ╱ 550
        ┌──────────────────────────────────────────────┐
        │  Perform blocks 510, 520, 530, 540           │
        │  on each of the two sub-matrices             │
        └──────────────────────────────────────────────┘
                               │
                               ▼              560
                  No     ╱ Termination ╲
                ◀──────◇    reached      ◇
                        ╲      ?       ╱
                               │ Yes
```

FIG. 6

600

|  | F1 | • • • | Fj | • • • | Fm |
|---|---|---|---|---|---|
| S1 | 0 | | 0 | | 1 |
| S2 | 0 | | 1 | | 0 |
| S3 | 0 | | 0 | | 0 |
| Sn | 0 | | 1 | | 0 |

Column Fj

FIG. 7

|  |  | 1 | 0 |
|---|---|---|---|
| Column Fk | 1 | a | b |
|  | 0 | c | d |

FIG. 8

600

800

| | F1 | • • • | Fj | • • • | Fm |
|---|---|---|---|---|---|
| S1 | | | | | |
| Sn | | | | | |

## FIG. 3B

|      | F1  | F2  | F3  | F4  | • • • | Fm-1 | Fm  |
|------|-----|-----|-----|-----|-------|------|-----|
| S1   | 2.5 | 0   | 0   | 0   |       | 0    | 0   |
| S2   | 0   | 0   | 7.2 | .01 |       | 0    | 0   |
| S3   | 0   | 6.1 | 0   | 0   |       | .2   | 0   |
| ⋮    |     |     |     |     |       |      |     |
| Sn-1 | 0   | 0   | 0   | 0   |       | 0    | 0   |
| Sn   | 0   | 0   | 6.5 | 0   |       | 0    | 1.2 |

## FIG. 9

## FIG. 10A

```
                              n=            96
                              u=          0.10
                              s=          0.31
                             rP=   6.39E·018
                             aP=   2.57E·014

                         N
```

```
NO                                      YES
GENE3754X                               GENE3754X

   n=            86                        n=           10
   u=          0.01                        u=          0.9
   s=          0.11                        s=          0.3
  rP=   3.60E·020
  aP=   1.45E·016                       N2

N1
```

```
NO                                      YES
GENE3753X                               GENE3753X

   n=            85                        n=            1
   u=     0.00000·                         u=     1.00000
   s=     0.00000·                         s=     0.00000


N11                                     N12
```

# FIG. 10B

N

```
n=          96
u=          0.10
s=          0.31
rP=  1.44E-010
aP=  5.78E-007
```

NO
GENE3941X

```
n=          86
u=          0.03
s=          0.18
rP=  6.33E-009
aP=  2.55E-005
```

N1

YES
GENE3941X

```
n=          10
u=          0.7
s=          0.5
```

N2

NO
GENE3565X

```
n=          79
u=     0.00000
s=     0.00000
```

N11

YES
GENE3565X

```
n=           7
u=          0.4
s=          0.5
```

N12